(19) Europäisches Patentamt
European Patent Office
Office européen des brevets

(11) EP 1 562 569 B1

(12) # EUROPEAN PATENT SPECIFICATION

(45) Date of publication and mention of the grant of the patent:
**23.12.2009 Bulletin 2009/52**

(21) Application number: **03767675.6**

(22) Date of filing: **24.10.2003**

(51) Int Cl.:
***A61K 31/155*** *(2006.01)* ***A61K 31/496*** *(2006.01)*
***A61K 31/4196*** *(2006.01)*

(86) International application number:
**PCT/EP2003/013335**

(87) International publication number:
**WO 2004/037239 (06.05.2004 Gazette 2004/19)**

(54) **ANTIFUNGAL MEDICAMENTS COMPRISING ARYLAMIDINE DERIVATIVES**
FUNGIZIDE MEDIKAMENTE ENTHALTEND ARYLAMIDINDERIVATE
MEDICAMENTS ANTIFONGIQUES CONTENANT DES DERIVES D'ARYLAMIDINE

(84) Designated Contracting States:
**AT BE BG CH CY CZ DE DK EE ES FI FR GB GR HU IE IT LI LU MC NL PT RO SE SI SK TR**

(30) Priority: **24.10.2002 EP 02356210**

(43) Date of publication of application:
**17.08.2005 Bulletin 2005/33**

(73) Proprietor: **Bayer CropScience S.A.**
**69009 Lyon (FR)**

(72) Inventors:
- **VORS, Jean-Pierre**
**F-69009 LYON (FR)**
- **O'NEILL, Elizabeth**
**F-69300 CALUIRE (FR)**
- **LABOURDETTE, Gilbert**
**F-71600 PARAY LE MONIAL (FR)**
- **MANSFIELD, Gillian**
**F-69004 LYON (FR)**
- **PILLMOOR, John**
**YORK , Yorkshire YO195PF (GB)**
- **BARCHIETTO, Thierry**
**F-95380 PUISEUX EN FRANCE (FR)**

(56) References cited:
**EP-A- 1 178 038 WO-A-00/46184**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 562 569 B1

## Description

The subject of the present invention relates to novel antifungal medicaments.

More precisely, the subject of the present invention concerns novel antifungal medicaments based on $N_2$-phenylamidine derivatives used for treating *Candida albicans* or *Aspergillus fumigatus* human infections, and optionally at least one other synergistic antifungal agent.

International application WO-00/46184 describes one or more $N_2$-phenylamidine derivatives. Such compounds are used in the agricultural field as antifungal agents.

The applicant has demonstrated quite unexpectedly that $N_2$-phenylamidine derivatives also constituted antifungal compounds of choice for the treatment of *Candida albicans* or *Aspergillus fumigatus* human infections.

Accordingly, one of the main objectives of the present invention is to provide a novel antifungal medicament based on $N_2$-phenylamidine derivatives.

Another main objective of the invention is to provide a completely effective novel antifungal medicament, especially as regards its efficacy against fungi.

Another main objective of the invention is to provide a novel fungicidal medicament synergistically combining at least one $N_2$-phenylamidine derivative and at least one other compound known as having an antifungal activity in human being.

Another main objective of the invention is to provide a novel antifungal medicament as defined in the above objectives and which is useful in the preventive and curative treatment of fungal diseases, in particular *Candida albicans* and *Aspergillus fumigatus* infections.

All these objectives, among others, have been achieved by the inventors who have had the merit of finding that $N_2$-phenylamidine derivatives surprisingly and unexpectedly exhibited a very high and perennial antifungal efficacy against *Candida albicans* or *Aspergillus fumigatus* human infections.

The present invention, therefore relates firstly to the use, for the manufacture of a medicament for treating *Candida albicans* or *Aspergillus fumigatus* human infections, of at least one compound of formula (I):

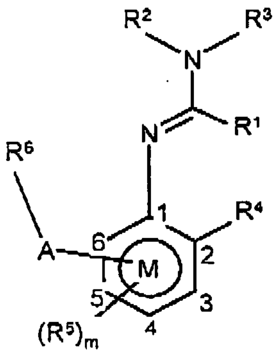

(I)

wherein:

- ° $R^1$ is selected from the group consisting of hydrogen, substituted or unsubstituted alkyl, substituted or unsubstituted alkenyl, substituted or unsubstituted alkynyl, and substituted or unsubstituted carbocyclic or heterocyclic monovalent group;
- ° $R^2$ and $R^3$ are independently selected from the group consisting of $R^1$ a cyano; an acyl; -ORa or -SRa, wherein Ra is selected from the group consisting of a substituted or unsubstituted alkyl, a substituted or unsubstituted alkenyl, a substituted or unsubstituted alkynyl, and a substituted or unsubstituted carbocyclic or heterocyclic monovalent group; or
- ° $R^2$ and $R^3$, or $R^2$ and $R^1$ form together and with the atoms linking them, a substituted or unsubstituted ring;
- ° $R^4$ is selected from the group consisting of a substituted or unsubstituted alkyl, a substituted or unsubstituted alkenyl, a substituted or unsubstituted alkynyl, a substituted or unsubstituted carbocyclic or heterocyclic monovalent group, hydroxyl, mercapto, azido, nitro, halo, cyano, unsubstituted or substituted acyl, amino, cyanato, thiocyanato, -SF5, -ORa, -SRa, and -Si(Ra)3
- ° m=0,1,2 or 3;
- ° the optional $R^5$ group or the optional $R^5$ groups, which may be mutually identical or different, have the same

definition as that given above for $R^4$;
° $R^6$ is an unsubstituted or substituted carbocyclic or heterocyclic group; and
° A is selected from the group consisting of a direct bond, -O-, -S(O)n-, -NR9-, -CR7=CR7-,- C/C-, -A1-, -A1-A1, -O-(A1)k-O-, -O-(A1)k-, -A3-, -A4-, -A1O-, -A1S(O)n-, -A2-, OA2-, NR9A2-, -OA2-A1-, -OA2-C(R7)=C(R8)- -S(O)nA1-, -A1-A4-, -A1-A4-C(R8)= N-N=CR8-, -A1-A4-C(R8)=N-X2-X3- , -A1-A4-A3-, -A1-A4-N(R9)-, -A1-A4-X-CH2-, -A1-A4-A1-, - A1-A4-CH2X-, -A1-A4-C(R8) =N-X2-X3-X1-, -A1-X-C(R8)=N-, -A1-X-C(R8)-N-N=CR8-, -A1-X-C(R8)=N-N(R9)-, -A1-X-A-X1-, -A1-O-A3-, -A1-O-C(R7)=C(R8)-, -A1-O-N(R9)-A2-N-(R9)-, -A1-O-N(R9)-A2-, -A1-N(R9)-A2-N(R9)-, -A1-N(R9)-A2-, -A1-N(R9)-N=C (R8)-, -A3-A1-, -A4-A3-, -A2-NR9-, -A1-A2-X1-, -A1-A1-A2-X1-, -O-A2-N(R9)-A2-, -CR7=CR7-A2-X1-, -C/C-A2-X1-, -N=C(R8)-A2-X1-, -C(R8)=N-N=C(R8)-, -C(R8)=N-N(R9)-, -(CH2)2-O-N=C(R8) and -X-A2-N(R9)-
wherein
n = 0, 1 or 2,
k = 1 to 9,
91 of 18
A1 = -CHR7-,
A2 = -C(=X)-,
A3 = -C(R8)=N-O-,
A4 = -O-N=C(R8)-,
X= O or S,
X1 = O, S, NR9 or a direct bond,
X2 = O, NR9 or a direct bond,
X3 = hydrogen, -C(=O)-, -S02- or a direct bond,
each R7 is independently selected from the group consisting of unsubstituted or substituted alkyl, substituted or unsubstituted cycloalkyl, substituted or unsubstituted phenyl, hydrogen, halogen, cyano, and acyl;
each R8 is independently selected from the group consisting of alkyl, substituted or unsubstituted alkenyl, substituted or unsubstituted alkynyl, substituted or unsubstituted alkoxy, substituted or unsubstituted alkylthio, a substituted or unsubstituted carbocyclic or heterocyclic monovalent group, and hydrogen;
each R9 is independently selected from the group consisting of unsubstituted or substituted alkyl, a substituted or unsubstituted monovalent carbocyclic or heterocyclic group, and acyl; or two R9 groups may form together, and with the atoms linking them, a 5-7-membered ring;
° the group represented on the right side of the bond A is linked to $R^6$; or -A-$R^6$ and $R^5$ form together with the benzene ring M, a system of unsubstituted or substituted condensed rings;
and optical and/or geometric isomers, tautomers and salts of (I) with an acid or a base that are pharmaceutically acceptable;
and mixtures thereof.

In the definitions of the compounds of formula (I) set out above, the various radicals and chemical terms used have, unless otherwise stated, the following meanings:

- "alkyl or alkyl-" means a linear or branched, saturated hydrocarbon radical containing from 1 to 8 carbon atoms;
- "alkenyl" means a linear or branched hydrocarbon radical containing from 1 to 8 carbon atoms and at least one unsaturation in the form of a double bond;
- "alkynyl" means a linear or branched hydrocarbon radical containing from 1 to 8 carbon atoms and at least one unsaturation in the form of a triple bond;
- "alkoxy" means an alkyloxy radical;
- "acyl" means the formyl radical or an alkoxycarbonyl radical;
- "cycloalkyl" means a saturated cyclic hydrocarbon radical containing from 3 to 8 carbon atoms;
- "haloalkyl" or "haloalkyl-" means a linear or branched, saturated hydrocarbon radical containing from 1 to 8 carbon atoms and substituted with one or more halogen atoms, in particular fluorine, chlorine and bromine;
- "aryl" means one or more aromatic radicals, preferably a phenyl or a naphthyl;
- "heterocycle" means an unsaturated or a completely or partially saturated cyclic radical containing from 3 to 8 atoms, chosen from carbon, nitrogen, sulphur and oxygen, for example, and without limitation, pyridyl, pyridinyl, quinolyl, furyl, thienyl, pyrrolyl, oxazolinyl;
- the expression "unsubstituted or substituted" means that the radicals thus termed may be substituted with one or more radicals chosen from chlorine, bromine, fluorine, iodine, alkyl, alkoxy, hydroxyl, nitro, amino; cyano and acyl.

According to a preferred embodiment of the invention, the products (I) correspond to formula (I) in which:

° $R^1$ is selected from the group consisting of hydrogen, substituted or unsubstituted alkyl, substituted or unsubstituted alkenyl, and substituted or unsubstituted alkynyl, wherein when $R^1$ is substituted alkyl, substituted alkenyl, or substituted alkynyl, the substituent(s) thereof is (are) selected from the group consisting of alkoxy, haloalkoxy, alkylthiol, halogen, unsubstituted phenyl, and phenyl substituted with a moiety selected from the group consisting of alkyl, haloalkyl, alkoxy, haloalkoxy, alkylthiol, and halogen;
° $R^2$ and $R^3$ are independently selected from the group consisting of R1, alkoxy, alkoxyalkyl, benzyloxy, cyano, and alkylcarbonyl;
° $R^4$ is selected from the group consisting of:

(a) substituted or unsubstituted alkyl, substituted or unsubstituted alkenyl, and substituted or unsubstituted alkynyl, wherein when $R^4$ is substituted alkyl, substituted alkenyl, or substituted alkynyl, the substituent(s) thereof is (are) selected from the group consisting of an alkoxy, haloalkoxy, alkylthiol, a halogen, unsubstituted phenyl, and phenyl substituted with a moiety selected from the group consisting of alkyl, haloalkyl, alkoxy, haloalkoxy, alkylthiol, and halogen;
(b) hydroxyl;
(c) halogen;
(d) cyano;
(e) acyl, amine, monoalkylamine, dialkylamine, unsubstituted phenyl, and phenyl substituted with a moiety selected from the group consisting of alkyl, haloalkyl, alkoxy,

haloalkoxy, and alkylthiol;
° m 0 or1;
° when it is present, $R^5$ is a group having the same definition as that given above for $R^4$,
° A is a direct bond, -O- , -S-, -NR9-, -CHR7- or -O-CHR7-,
° each R9, when any are present, is selected from the group consisting of hydrogen, substituted or unsubstituted alkyl, substituted or unsubstituted alkenyl, and substituted or unsubstituted alkynyl, wherein when an R9 is a substituted alkyl, a substituted alkenyl, or a substituted alkynyl, the substitutent(s) thereof is (are) selected from the group consisting of alkoxy, haloalkoxy, alkylthiol, halogen, unsubstituted phenyl, and phenyl substituted with a moiety selected from the group consisting of alkyl, haloalkyl, alkoxy, haloalkoxy, alkylthiol, and halogen;
° R7 is selected from the group consisting of R9; hydroxyl; halogen; cyano; acyl; alkoxy;
° haloalkoxy; and alkylthiol;
A is linked to the 4-position of the benzene ring M; and
° $R^6$ is a substituted or unsubstituted phenyl or an aromatic heterocycle which when $R^6$ is a substituted phenyl or substituted aromatic heterocycle, the substituent(s) thereof is (are) selected from the group consisting of
(a) hydroxyl;
(b) halogen;
(c) cyano;
(d) acyl;
(e) amine;
(f) alkylamine;
(g) dialkylamine;
(h) alkyl;
(i) haloalkyl;
(j) RaO-alkyl;
(k) acyloxyalkyl;
(l) cyanooxyalkyl;
(m) alkoxy;
(n) haloalkoxy;
(o) alkylthiol;
(p) cycloalkyl unsubstituted or substituted with a moiety selected from the group consisting of alkyl, haloalkyl, alkoxy, haloalkoxy, and alkylthiol; and
(q) benzyl unsubstituted or substituted with a moiety selected from the group consisting of alkyl, haloalkyl, alkoxy, haloalkoxy, and alkylthiol.

The compounds of formula (I) which are still more especially preferred are those possessing the following

characteristics:

- $R^1$ represents an hydrogen atom
- $R^2$, $R^3$, $R^4$, and $R^6$ independently represent $C_1$-$C_6$ alkyl and $R^5$ is linked to the carbon at $C_5$ of the benzyl ring M, with m =1;
- A is linked to the carbon at $C_4$ of the benzyl ring M and represents-O-;
- $R^6$ represents unsubstituted aryl or aryl substituted with at least one alkyl and/or with at least one halogen.

The compounds of formula (I) which are even still more especially preferred are those possessing the following characteristics:

- $R^1$ represents an hydrogen atom ;
- $R^2$ represents ethyl;
- $R^3$, $R^4$ and $R^5$ represent methyl and $R^6$ is linked to the carbon at $C_5$ of the benzyl ring M, with m = 1;
- A is linked to the carbon at $C_4$ of the benzyl ring M and represents-O-;
- $R^6$ represents benzyl substituted with at least one alkyl and/or with at least one halogen.

By way of example, the compounds (I) used are, inter alia:

- *N*-ethyl-*N*-methyl-N-[4-(4-chloro-3-trifluoromethylphenoxy)-2,5-dimethyl phenyl]imidoformamide,
- and/or *N*-ethyl-*N*-methyl-*N'*-[4-(4-fluoro-3-trifluoromethylphenoxy)-2,5-dimethylphenyl]imidoformamide,
- and/or *N*-ethyl-*N*-methyl-*N'*-[4-(4-cyano-3-trifluoromethylphenoxy)-2,5-dimethylphenyl]imidoformamide,
- and the possible tautomers and salts, in particular addition salts with an acid or a base, which are pharmaceutically acceptable, of these compounds (I).

According to an advantageous embodiment of the invention, the antifungal medicament comprises at least one other antifungal compound (II).

Such an antifungal compound forms part of the compounds known to persons skilled in the art and is advantageously chosen from the following families of compounds:

- azoles, such as bifonazole, butoconazole, clotrimazole, eberconazole, econazole, fenticonazole, fluconazole, itraconazole, ketoconazole, miconazole, oxiconazole, posaconazole, sulconazole, terconazole, tioconazole, voriconazole, zinoconazole;
- polyenes, such as amphotericin B, nystatin;
- allylamines and benzylamines, such as butenafine, naftifine, terbinafine;
- thiocarbamates, such as tolnaftate;
- candins, such as caspofungin, cilofungin;
- nucleoside analogues, such as flucytosine;
- sordarins;
- polyoxines and nikkomycins, such as nikkomycins Z, J, pseudo J, PX, RZ, pseudo Z;
- pradimicins, such as pradimicin A;
- benanomycins;
- aureobasidins;
- UK-2A or UK-3A;
- cationic peptides;

taken alone or as a mixture, and their possible tautomers and salts, in particular addition salts with an acid or a base, their lipid or liposomal formulations, which are pharmaceutically acceptable.

From the point of view of weight, it should be specified that in accordance with the invention, the mass ratio (I/II) is defined as follows:

| | | |
|---|---|---|
| | 0.02 | $\leq$ II/I $\leq$ 50 |
| preferably | 0.1 | $\leq$ I/II $\leq$ 20 |
| and still more preferably | 0.5 | $\leq$ III $\leq$ 10. |

In the case where compound (II) is fluconazole or itraconazole (or one of their equivalents), it has been found that the mass ratio (I/II) is advantageously between 0.5 and 10.

The compound (I)/compound (II) ratio is defined as being the ratio by weight of these 2 compounds. The same applies to any ratio of 2 chemical compounds, which is subsequently measured in the present text, since a definition different from this ratio is not expressly given.

In the compositions according to the invention, the compound (I)/compound (II) ratio is advantageously chosen so as to produce a synergistic effect. The term synergistic effect, as understood in the present text, is defined in the examples at point 2.4.

As is evident from the preceding text, the preferred examples of synergistic combinations according to the invention will comprise compound (I), fluconazole and/or itraconazole, and their possible tautomers and addition salts with an acid or a base, as long as these equivalents are acceptable in the human or veterinary pharmaceutical field.

The compound (I)/compound (II) ratio ranges indicated above do not in any way limit the scope of the invention, but are rather mentioned as a guide, persons skilled in the art being entirely capable of carrying out additional trials in order to find other values of the apportioning ratio of these two compounds, for which a synergistic effect is observed.

According to a preferred feature of the invention, the quantity of active agents (I/II) present in the fungicidal compositions according to the invention is between 0.5 and 99% by weight

Naturally, the antifungal medicaments according to the invention based on at least one compound (I) and at least one compound (II) may also comprise one or more other active products.

In addition to these additional active agents, the antifungal medicaments according to the invention may also contain any other excipient and/or auxiliary agent useful in pharmaceutical formulations.

As regards the presentations of the medicaments according to the invention, it should be indicated that they are appropriate for all known and suitable galenic forms in antifungal treatment Thus, these medicaments may be provided in the form of formulations for administration orally, topically, intravenously or intraperitoneally.

As regards the preparation of compounds (I), reference may be made to international patent application WO-00/46184.

In the case of the preparation of the known synergistic compounds (II), these are prepared according to the usual pharmacopoea rules.

According to another of its objects, the invention relates to a method for controlling curatively or preventively, human or animal pathogenic fungi, characterized in that it consists in using an antifungal medicament as defined above.

The antifungal medicaments according to the invention usually contain from 0.5 to 99% of the combination of compound (I) and compound (II).

The optimum dose quite obviously depends on the type of pathogenic fungus to be treated and the seriousness of the infection.

The following examples are given purely by way of illustration of the invention and do not limit it in any way.

## EXAMPLES

## IN VITRO MEASUREMENTS OF THE ANTIFUNGAL ACTIVITY OF VARIOUS COMPOUNDS USED ALONE OR IN COMBINATION AGAINST *CANDIDA ALBICANS* AND *ASPERGILLUS FUMIGATUS*

### 1 - OBJECTIVE OF THE TRIALS

The objective of the trials is to test the efficacy of a compound of the arylamidine type, and two antifungal compounds of the family of azoles, fluconazole and itraconazole, already commercially available. These trials are aimed, in the first instance, at comparing the antifungal activity of the arylamidine type compound, taken alone, with that of azoles. Their aim is also to demonstrate the synergistic properties of the combinations of such compounds.

### 2 - MATERIALS AND METHODS

#### 2.1 - Strains and media:

The following fungi were used for this study: *Candida albicans* strains IP 48.72 (ATCC 10231) and *Aspergillus fumigatus* strain IP 864.64 obtained from the Collection Nationale de Cultures de Microorganismes (CNCM) of the Institut Pasteur. The strains are cultured on Yeast Extract-Peptone-Dextrose (YEPD) agar medium comprising 0.5% yeast extract, 0.5% bactopeptone, 2% glucose and 2% agar at 30°C and in the dark.

#### 2.2 - The products tested are:

COMPOUND (I.1)- *N*-ethyl-*N*-methyl-*N*'-[4-(4-chloro-3-trifluoromethylphenoxy)-2,5-dimethylphenyl]imidoformamide.

→ COMPOUND (I.2): N-ethyl-N-methyl-N-[4-(4-cyano-3-trifluoromethylphenoxy)-2,5-dimethylphenyl]imidoformamide.
→ COMPOUND (II.1): fluconazole.
→ COMPOUND (II.2): itraconazole.

All these compounds were prepared in a DMSO solution at a final concentration of 100 mg/ml. The stock solutions are stored at -20°C up to the time of use.

**2.3 - Trial medium:**

The trials are carried out in RPMI 1640 medium with no sodium bicarbonate, but with L-glutamine buffered with 0.165 mol per litre of 3-[N-morpholino]propanesulphonic acid (MOPS), enriched («rich») or otherwise («minimal») with 2% glucose. The pH of this medium is adjusted to 7.0. The medium is sterilized by filtration (0.22 μm) and stored at 4°C up to the time of use.

**2.4- Measurement on microtitre plates:**

All the antifungal tests are carried out on microtitre plates. The initial suspensions of spores are prepared in a sterile solution containing 0.85% NaCl, supplemented with Tween 80 at 0.01%. These initial suspensions are then diluted in the culture medium (RPMI 1640 enriched or otherwise with 2% glucose) to a final concentration of $10^4$ spores per ml. The measurements of viability of each inoculum are verified by subcultures of a volume of 300 μl on YEPD agar medium.
The antifungal compounds are tested in a range of concentrations ranging from 0.026 to 100 μg of active ingredient/ml. These antifungal compounds are then diluted in RPMI 1640 medium enriched or otherwise with 2% glucose. A final DMSO concentration of 0.2% is used throughout the measurements. Each trial is carried out on a series of dilutions of antifungal compounds, in duplicate. The antifungal dilutions (0.1 ml) and the fungal inoculum (0.1 ml) are added to each of the wells of the microtitre plate. The plates are then read with a spectrophotometer (ELX 800UV Bio-Tek Instruments, Inc) at a wavelength of 590 nm. The plates are read immediately after (t = 0) and after 48 hours' incubation at 30°C and in the dark. The optical density values obtained are correlated with fungal growth between the times t = 0 and t = 48 hours. The optical density values are used to calculate the percentage inhibition of growth for each concentration of antifungals by comparison with the control. The values are then used to plot a dose-response curve and the $EC_{50}$ value is determined for each fungus and each compound with the aid of the Grafit 5.0® software (Erithacus software Ltd).
The method which was used to measure the type of interaction existing between the antifungal compounds in the form of mixtures is the Wadley method.
In the Wadley approach, the dose-response curve for each of the compounds A and B, and for the mixture AB, is constructed. The $EC_{50}$ is calculated for each compound taken individually and for the mixture. If a and b are the absolute quantities of the compounds A and B in the mixture (a=1, b=1, a + b = 2 under our conditions), the expected effective concentration ($EC_{50exp}$) may be calculated in the following manner:

$$EC_{50exp} = (a + b)/[a/EC_{50A}) + b/(EC_{50B})$$

The Wadley approach may be used to estimate the type of interaction existing between the fungal compounds, regardless of their concentration. Its reliability is not dependent on the percentage inhibition. The type of interaction between two compounds is given by the level of interaction (LI) which corresponds to the ratio between the expected effective concentration ($EC_{50exp}$) and that observed ($EC_{50obs}$) of the mixture. The nature of the interaction obtained by the Wadley formula is presented in Table 1 (see U. Gisi, Synergistic interaction in fungicide mixtures, 1896. Phytopathology 86, 1273-1279) below.

| Level of interaction | Mathematical definition | Biological definition |
|---|---|---|
| <1 | Antagonist | |
| 1 | | |
| >1 | Additive | |
| <0.5 | | Antagonist |
| 0.5 - 1 | Synergistic | Additive |

(continued)

| Level of interaction | Mathematical definition | Biological definition |
|---|---|---|
| >1.5 | | Synergistic |

## 3-RESULTS

**Table 1:** Efficacy *in vitro* of compound I.1, of compound II.1 and of compound II.2 used alone against *Aspergillus fumigatus* cultured on minimal RPMI 1640 medium (MM).

| % inhibition$^{\alpha}$ | Dose (μg/ml) | | | | | | |
|---|---|---|---|---|---|---|---|
| | 0.026 | 0.098 | 0.39 | 1.562 | 6.25 | 25 | 100 |
| Compound I.1 | 1 | 57.9 | 80.1 | 94.6 | 97.1 | 98.4 | 98.1 |
| Compound II.1 | 4.8 | 4.8 | 5.8 | 8.7 | 7.7 | 23.1 | 41.4 |
| Compound II.2 | 2.2 | 31.8 | 49.3 | 99.6 | 100 | 100 | 99 |
| α The percentage inhibition of growth is determined after 48 hours' incubation at 30°C and in the dark. | | | | | | | |

**Table 2**: Efficacy *in vitro* of compound I.1, of compound II.1 and of compound II.2 used alone against *Aspergillus fumigatus* cultured on rich RPMI 1640 medium (RM).

| % inhibition$^{\alpha}$ | Dose (μg/ml) | | | | | | |
|---|---|---|---|---|---|---|---|
| | 0.026 | 0.098 | 0.39 | 1.562 | 6.25 | 25 | 100 |
| Compound I.1 | 2.24 | 23.1 | 51.1 | 97.4 | 98.1 | 98.1 | 97.4 |
| Compound II.1 | 11.2 | 20.7 | 25.9 | 27.6 | 28.4 | 48.3 | 46.6 |

(continued)

| % | Dose (μg/ml) | | | | | | |
|---|---|---|---|---|---|---|---|
| Compound 11.2 | 18.6 | 47.5 | 88.6 | 99.2 | 100 | 100 | 100 |
| α The percentage inhibition of growth is determined after 48 hours' incubation at 30°C and in the dark. | | | | | | | |

**Table 3**: Efficacy *in vitro* of compound I.1, of compound II.1 and of compound II.2 used alone against *Candida albicans* cultured on minimal RPMI 1640 medium (MM).

| % | Dose (μg/ml) | | | | | | |
|---|---|---|---|---|---|---|---|
| inhibition$^{\alpha}$ | 0.026 | 0.098 | 0.39 | 1.562 | 6.25 | 25 | 100 |
| Compound I.1 | 4.6 | 7 | 46.1 | 96.2 | 98.7 | 99.2 | 98.1 |
| Compound II.1 | 2.1 | 9.7 | 38.5 | 64.7 | 77.7 | 81.9 | 91.6 |
| Compound II.2 | 58.1 | 75.4 | 78.2 | 74.6 | 78.6 | 87.9 | 95.2 |
| α The percentage inhibition of growth is determined after 48 hours' incubation at 30°C and in the dark. | | | | | | | |

**Table 4:** Efficacy in vitro of compound I.1, of compound II.1 and of compound II.2 used alone against *Candida albicans* cultured on rich RPMI 1640 medium (RM).

| % | Dose (μg/ml) | | | | | | |
|---|---|---|---|---|---|---|---|
| inhibition $^{\alpha}$ | 0.026 | 0.098 | 0.39 | 1.562 | 6.25 | 25 | 100 |
| Compound I.1 | 12.6 | 27.3 | 43.6 | 96.9 | 99.5 | 99.5 | 98.8 |

(continued)

| % | Dose (μg/ml) | | | | | | |
|---|---|---|---|---|---|---|---|
| Compound II.1 | 11.1 | 32.5 | 72.6 | 86.8 | 94 | 95.3 | 36.1 |
| Compound II.2 | 44.7 | 85.1 | 86.2 | 85.1 | 86.2 | 95.2 | 95.2 |
| α The percentage inhibition of growth is determined after 48 hours' incubation at 30°C and in the dark. | | | | | | | |

**Table 5**: Efficacy[α] *in vitro* of compound I.1, of compound II.1 and of compound II.2 used alone against *Aspergillus fumigatus* and *Candida albicans* cultured on minimal RPMI 1640 medium (MM) and on rich RPMI 1640 medium (RM).

| Fungicide | $EC_{50}$ (μg/ml) | | | |
|---|---|---|---|---|
| | *Aspergillus fumigatus* | | *Candida albicans* | |
| | MM | RM | MM | RM |
| Compound I.1 | 0.1 | 0.31 | 0.41 | 0.3 |
| Compound II.1 | 575[β] | 125[β] | 0.96 | 0.19 |
| Compound II.2 | 0.27 | 0.27 | 0.022 | 0.017 |
| α The percentage inhibition of growth is determined after 48 hours' incubation at 30°C and in the dark.<br>β These $EC_{60}$ values are extrapolated from the analysis of the dose-reponse curves obtained with the aid of the Grafit 5.0® software. | | | | |

**Table 6**: Evaluation *in vitro* of the extent of the interaction between compound I.1, compound II.1 and compound II.2 using the Wadley formula against *Aspergillus fumigatus* and *Candida albicans* cultured on minimal RPMI 1640 medium (MM) and rich RPMI 1640 medium (RM).

| Fungicide | *Aspergillus fumigatus* | | *Candida albicans* | |
|---|---|---|---|---|
| | MM | RM | MM | RM |
| Mixture I.1 + II.1 | | | | |
| $EC_{50\ obs}$ | 0.200 | 0.122 | 0.225 | 0.451 |
| $EC_{50\ exp}$ | 0.21 | 0.62 | 0.573 | 0.223 |
| L.I.[α] | 1.06 | 5.09 | 2.54 | 0.494 |
| Mixture I.1 + II.2 | | | | |
| $EC_{50obs}$ | 0.270 | 0.175 | 0.112 | 0.129 |
| $EC_{50exp}$ | 0.15 | 0.292 | 0.042 | 0.032 |
| L.I. | 0.56 | 1.67 | 0.37 | 0.25 |

(continued)

| Fungicide | *Aspergillus fumigatus* | | *Candida albicans* | |
|---|---|---|---|---|
| | MM | RM | MM | RM |
| | | | 6 | |
| α The level of interaction (L.I.) corresponds to the ratio of the expected effective concentration ($EC_{50exp}$) to the observed effective concentration ($EC_{50obs}$) of the mixture. The synergistic interaction is present when the level of interaction is greater than 1.5 (values in bold). | | | | |

**4 - CONCLUSIONS**

The various results obtained and presented above demonstrate the efficacy of compound I.1, whether on minimal RPMI 1640 medium (MM) or on rich medium (RM), against *Aspergillus fumigatus* and *Candida albicans* with $EC_{50}$ values between 0.1 and 0.5 pglml, and therefore having an activity equivalent to that of itraconazole (compound II.2) against *Aspergillus fumigatus* and an activity at least equivalent to that of fluconazole (compound II.1) against *Candida albicans.*

As regards the interactions between compounds, the results obtained by the Wadley method show that the combination of compound I.1 and fluconazole (compound II.1) exhibits surprising synergistic effects both on *Aspergillus fumigatus* and on *Candida albicans.* The antifungal medicament according to the invention therefore constitutes real progress in terms of improvement of the antifungal activity compared with the references on the market.

During a second study, compound I.2 according to the invention was tested. It is: *N*-ethyl-*N*-methyl-*N'*-[4-(4-cyano-3-trifluoromethylphenoxy)-2,5-dimethylphenyl]imidoformamide.

Compound (I.1) according to the invention and fluconazole (II.1) and itraconazole (II.2) were also tested *in vitro* on *Candida albicans* and *Aspergillus fumigatus* in enriched medium.

The experimental conditions are the same as previously described.

**Results**

**Table 1**. Efficacy$^{\alpha}$ *in vitro* of compounds I.1, I.2, II.1 and II.2. used alone against *Candida albicans* and *Aspergillus fumigatus* in RPMI 1640 medium enriched with 2% glucose.

| Fungicide | $EC_{50}{}^{\chi}$ (μg/ml) | |
|---|---|---|
| | *Candida albicans* | *Aspergillus fumigatus* |
| | IP 48.72$^{\beta}$ | IP 864.64$^{\beta}$ |
| Compound I.1 | 0.635 | 0.179 |
| Compound I.2 | 0.205 | 0.088 |
| Compound II.1 | 0.354 | 125 |
| Compound II.2 | 0.082 | 0.187 |
| $^{\alpha}$The percentage inhibition of growth is determined after 48 hours of incubation at 30°C and in the dark.<br>$^{\beta}$ Strains IP48.72 and IP864.64 served as references for these experiments.<br>$^{\chi}$ These $EC_{50}$ values are determined after analyses of the dose-reponse curves obtained by means of the Grafit 5.0® software. | | |

On *Candida albicans,* the $EC_{50}$ of compound I.1 is 1.8 and 7.7 times higher than that of compounds II.1 and II.2, whereas compound I.2 shows better efficacy *in vitro* against *Candida albicans* than compounds I.1 and II.1.

On *Aspergillus fumigatus* (IP 864.64), the $EC_{50}$ of compound I.1 is about 700 times lower than that of compound II.1 and similar to that of compound II.2, whereas compound I.2 shows efficacy *in vitro* on *Aspergillus fumigatus* twice higher than that of compounds I.1 and II.2.

## Claims

**1.** (amended) Use, for the manufacture of a medicament for treating *Candida albicans* or *Aspergillus fumigatus* human infections, of at least one compound of formula (I):

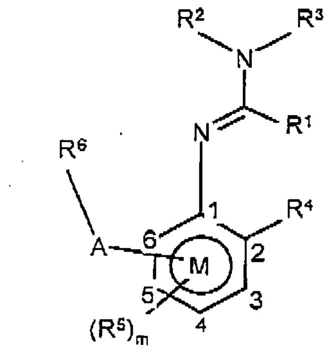

(I)

wherein:

° R' is selected from the group consisting of hydrogen, substituted or unsubstituted alkyl, substituted or unsubstituted alkenyl, substituted or unsubstituted alkynyl, and substituted or unsubstituted carbocyclic or heterocyclic monovalent group;
° $R^2$ and $R^3$ are independently selected from the group consisting of $R^1$; a cyano; an acyl; -ORa or -SRa, wherein Ra is selected from the group consisting of a substituted or unsubstituted alkyl, a substituted or unsubstituted alkenyl, a substituted or unsubstituted alkynyl, and a substituted or unsubstituted carbocyclic or heterocyclic monovalent group; or
° $R^2$ and $R^3$, or $R^2$ and $R^1$ form together and with the atoms linking them, a substituted or unsubstituted ring:
° $R^4$ is selected from the group consisting of a substituted or unsubstituted alkyl, a substituted or unsubstituted alkenyl, a substituted or unsubstituted alkynyl, a substituted or unsubstituted carbocyclic or heterocyclic monovalent group, hydroxyl, mercapto, azido, nitro, halo, cyano, unsubstituted or substituted acyl, amino, cyanato, thiocyanato, -SF5, -ORa, -SRa, and -Si(Ra)3
° m = 0,1, 2 or 3;
° the optional $R^5$ group or the optional $R^5$ groups, which may be mutually identical or different, have the same definition as that given above for $R^4$;
° $R^6$ is an unsubstituted or substituted carbocyclic or heterocyclic group; and
A is selected from the group consisting of a direct bond, -O-, -S(O)n-, -NR9-, -CR7=CR7-, -C/C-, -A1-, -A1-A1, -O-(A1)k-O-, -O-(A1)k-, -A3-, -A4-, -A1O-, -A1S(O)n-, -A2-, OA2-, -NR9A2-, -OA2-A1-, -OA2-C(R7)=C(R8)-, -S(O)nA1-, -A1-A4-, -A1-A4-C(R8)= N-N=CR8-,- A1-A4-C(R8)=N-X2-X3- , -A1-A4-A3-, -A1-A4-N(R9)-, -A1-A4-X-CH2-, -A1-A4-A1-, -A1-A4-CH2X-, -A1-A4-C(R8)=N-X2-X3-X1-, -A1-X-C(R8)=N-, -A1-X-C(R8)=N-N=CR8-, -A1-X-C(R8)=N-N(R9)-, -A1-X-A-X1-, -A1-O-A3-, -A1-O-C(R7)=C(R8)-, -A1-O-N(R9)-A2-N- (R9)-, -A1-O-N(R9)-A2-, -A1-N(R9)-A2-N(R9)-, -A1-N(R9)-A2-, -A1-N(R9)-N=C(R8)-, -A3-A1-, -A4-A3-, -A2-NR9-, -A1-A2-X1-, - A1-A1-A2-X1-, -O-A2-N(R9)-A2-, -CR7=CR7-A2-X1-, -C/C-A2-X1-, -N=C(R8)-A2-X1-, -C(R8)=N-N=C(R8)-, -C(R8)=N-N(R9)-, -(CH2)2-O-N=C(R8)- and -X-A2-N(R9)-
wherein
n = 0, 1 or 2,
k = 1 to 9,
11 of 18
A1 = -CHR7-,
A2 = -C(=X)-,
A3 = -C(R8)=N-O-,
A4 = -O-N=C(R8)-,
X=O or S.
X1 = O, S, NR9 or a direct bond,
X2 = O, NR9 or a direct bond,
X3 = hydrogen, -C(=O)-, -SO2- or a direct bond,

each R7 is independently selected from the group consisting of unsubstituted or substituted alkyl, substituted or unsubstituted cycloalkyl, substituted or unsubstituted phenyl, hydrogen, halogen, cyano, and acyl;
each R8 is independently selected from the group consisting of alkyl, substituted or unsubstituted alkenyl, substituted or unsubstituted alkynyl, substituted or unsubstituted alkoxy, substituted or unsubstituted alkylthio, a substituted or unsubstituted carbocyclic or heterocyclic monovalent group, and hydrogen;
each R9 is independently selected from the group consisting of unsubstituted or substituted alkyl, a substituted or unsubstituted monovalent carbocyclic or heterocyclic group, and acyl; or two R9 groups may form together, and with the atoms linking them, a 5-7-membered ring;
° the group represented on the right side of the bond A is linked to $R^6$; or -A-$R^6$ and $R^5$ form together with the benzene ring M, a system of unsubstituted or substituted condensed rings;

and optical and/or geometric isomers, tautomers and salts of (I) with an acid or a base that are pharmaceutically acceptable;
and mixtures thereof.

**2.** (amended) The use according to claim 1, **characterized in that**:

° $R^1$ is selected from the group consisting of hydrogen, substituted or unsubstituted alkyl, substituted or unsubstituted alkenyl, and substituted or unsubstituted alkynyl, wherein when $R^1$ is substituted alkyl, substituted alkenyl, or substituted alkynyl, the substituent(s) thereof is (are) selected from the group consisting of alkoxy, haloalkoxy, alkylthiol, halogen, unsubstituted phenyl, and phenyl substituted with a moiety selected from the group consisting of alkyl, haloalkyl, alkoxy, haloalkoxy, alkylthiol, and halogen;
° $R^2$ and $R^3$ are independently selected from the group consisting of R1, alkoxy, alkoxyalkyl, benzyloxy, cyano, and alkylcarbonyl;
° $R^4$ is selected from the group consisting of:

(a) substituted or unsubstituted alkyl, substituted or unsubstituted alkenyl, and substituted or unsubstituted alkynyl, wherein when $R^4$ is substituted alkyl, substituted alkenyl, or substituted alkynyl, the substituent(s) thereof is (are) selected from the group consisting of an alkoxy, haloalkoxy, alkylthiol, a halogen, unsubstituted phenyl, and phenyl substituted with a moiety selected from the group consisting of alkyl, haloalkyl, alkoxy, haloalkoxy, alkylthiol, and halogen;
(b) hydroxyl;
(c) halogen;
(d) cyano;
(e) acyl, amine, monoalkylamine, dialkylamine, unsubstituted phenyl, and phenyl substituted with a moiety selected from the group consisting of alkyl, haloalkyl, alkoxy, haloalkoxy, and alkylthiol;

° m = 0 or 1;
° when it is present, $R^5$ is a group having the same definition as that given above for $R^4$,
° A is a direct bond, -O-, -S-, -NR9-, -CHR7- or -O-CHR7-,
° each R9, when any are present, is selected from the group consisting of hydrogen, substituted or unsubstituted alkyl, substituted or unsubstituted alkenyl, and substituted or unsubstituted alkynyl, wherein when an R9 is a substituted alkyl, a substituted alkenyl, or a substituted alkynyl, the substitutent(s) thereof is (are) selected from the group consisting of alkoxy, haloalkoxy, alkylthiol, halogen, unsubstituted phenyl, and phenyl substituted with a moiety selected from the group consisting of alkyl, haloalkyl, alkoxy, haloalkoxy, alkylthiol, and halogen;
° R7 is selected from the group consisting of R9; hydroxyl; halogen; cyano; acyl; alkoxy;
° haloalkoxy; and alkylthiol;
A is linked to the 4-position of the benzene ring M; and
° $R^6$ is a substituted or unsubstituted phenyl or an aromatic heterocycle which when $R^6$ is a substituted phenyl or substituted aromatic heterocycle, the substituent(s) thereof is (are) selected from the group consisting of

(a) hydroxyl;
(b) halogen:
(c) cyano;
(d) acyl;
(e) amine;
(f) alkylamine;
(g) dialkylamine;

(h) alkyl;
(i) haloalkyl;
(j) RaO-alkyl;
(k) acyloxyalkyl;
(l) cyanooxyalkyl;
(m) alkoxy;
(n) haloalkoxy;
(o) alkylthiol;
(p) cycloalkyl unsubstituted or substituted with a moiety selected from the group consisting of alkyl, haloalkyl, alkoxy, haloalkoxy, and alkylthiol; and
(q) benzyl unsubstituted or substituted with a moiety selected from the group consisting of alkyl, haloalkyl, alkoxy, haloalkoxy, and alkylthiol.

**3.** (amended) The use according to Claim 1, **characterized in that**

- $R^1$ represents an hydrogen atom
- $R^2$, $R^3$, $R^4$, and $R^5$ independently represent $C_1$-$C_6$ alkyl and $R^5$ is linked to the carbon at $C_5$ of the benzyl ring M, with m = 1;
- A is linked to the carbon at $C_4$ of the benzyl ring M and represents-O-;
- $R^6$ represents unsubstituted aryl or aryl substituted with at least one alkyl and/or with at least one halogen.

**4.** (New) The use according to Claim 1, **characterized in that**:

- R' represents an hydrogen atom ;
- $R^2$ represents ethyl;
- $R^3$, $R^4$ and $R^5$ represent methyl and $R^6$ is linked to the carbon at $C_5$ of the benzyl ring M, with m=1;
- A is linked to the carbon at $C_4$ of the benzyl ring M and represents-O-;
- $R^6$ represents benzyl substituted with at least one alkyl and/or with at least one halogen.

**5.** (amended) The use according to claim 3 or 4, **characterized in that** compound (I) is selected from the group consisting of:

- *N*-ethyl-*N*-methyl-*N*-[4-(4-chloro-3-trifluoromethylphenoxy)-2,5dimethylphenyl]imidoformamide,
- *N*-ethyl-*N*-methyl-*N*-[4-(4-fluoro-3-trifluoromethylphenoxy)-2,5-dimethylphenyl]imidoformamide,
- *N*-ethyl-*N*-methyl-*N*'-[4-(4-cyano-3-trifluoromethylphenoxy)-2,5-dimethylphenyl]imidoformamide, and the possible tautomers and salts, in particular addition salts with an acid or a base, which are pharmaceutically acceptable, of these compounds (I).

**6.** (amended) The use according to one of claims 1 to 5, **characterized in that** the medicament further comprises at least one other antifungal compound (II).

**7.** (amended) The use according to claim 6, **characterized in that** the antifungal compound (II) is chosen from the group consisting of azoles; polyenes; allylamines, benzylamines; thiocarbamates; candins; nucleoside analogues; sordarins; polyoxines, nikkomycins; pradimicins; benanomycins; aureobasidins; UK-2A or UK-3A; cationic peptides; taken alone or as a mixture, and their possible tautomers and salts, in particular addition salts with an acid or a base, their lipid or liposomal formulations, which are pharmaceutically acceptable.

**8.** (new) The use according to claim 7, **characterized in that** the antifungal compound (II) is chosen from the group consisting of bifonazole, butoconazole, clotrimazole, eberconazole, econazole, fenticonazole, fluconazole, itraconazole, ketoconazole, miconazole, oxiconazole, posaconazole, sulconazole, terconazole, tioconazole, voriconazole, zinoconazole, amphotericin B, nystatin, butenafine, naftifine, terbinafine, tolnaftate, caspofungin, cilofungin, flucytosine, nikkomycins Z, J, pseudo J, PX, RZ, pseudo Z, pradimicin A;
taken alone or as a mixture, and their possible tautomers and salts, in particular addition salts with an acid or a base, their lipid or liposomal formulations, which are pharmaceutically acceptable.

**9.** (amended) The use according to claims 6 to 8, **characterized in that** the mass ratio (I/II) is $0.02 \leq I/II \leq 50$

**10.** (amended) The use according to one of claims 1 to 9 **characterized in that** the medicament further comprises at

least one pharmaceutically acceptable excipient.

**11.** (amended) The use according to one of claims 1 to 10, **characterized in that** the medicament comprises from 0.5 to 99% of the combination of compound (I) and compound (II),

## Patentansprüche

**1.** (geändert) Verwendung für die Herstellung eines Arzneimittels zur Behandlung von *Candida albicans*- oder *Aspergillus fumigatus*-Infektionen des Menschen von mindestens einer Verbindung der Formel (I):

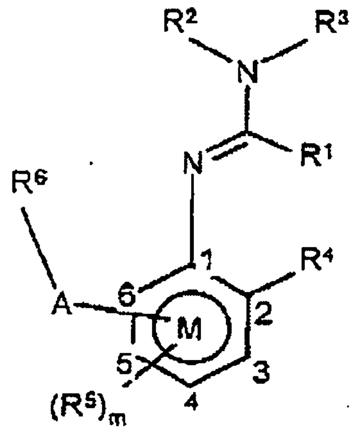

(I)

in der:

o $R^1$ ausgewählt ist aus der Gruppe Wasserstoff, substituiertes oder unsubstituiertes Alkyl, substituiertes oder unsubstituiertes Alkenyl, substituiertes oder unsubstituiertes Alkinyl und substituierte oder unsubstituierte carbocyclische oder heterocyclische einwertige Gruppe;
o $R^2$ und $R^3$ unabhängig ausgewählt sind aus der Gruppe $R^1$, Cyano, Acyl, -ORa oder -SRa, wobei Ra ausgewählt ist aus der Gruppe substituiertes oder unsubstituiertes Alkyl, substituiertes oder unsubstituiertes Alkenyl, substituiertes oder unsubstituiertes Alkinyl und substituierte oder unsubstituierte carbocyclische oder heterocyclische einwertige Gruppe; oder
o $R^2$ und $R^3$ oder $R^2$ und $R^1$ gemeinsam mit den sie verbindenden Atomen einen substituierten oder unsubstituierten Ring bilden;
o $R^4$ ausgewählt ist aus der Gruppe substituiertes oder unsubstituiertes Alkyl, substituiertes oder unsubstituiertes Alkenyl, substituiertes oder unsubstituiertes Alkinyl und substituierte oder unsubstituierte carbocyclische oder heterocyclische einwertige Gruppe, Hydroxy, Mercapto, Azido, Nitro, Halogen, Cyano, unsubstituiertes oder substituiertes Acyl, Amino, Cyanato, Thiocyanato, $-SF_5$, -ORa, -SRa und -Si(Ra)3,
o m = 0, 1, 2 oder 3;
o die fakultative $R^5$-Gruppe oder die fakultativen $R^5$-Gruppen, die jeweils identisch oder verschieden sein können, wie oben für $R^4$ definiert ist/sind;
° $R^6$ eine unsubstituierte oder substituierte carbocyclische oder heterocyclische Gruppe ist; und
° A ausgewählt ist aus der Gruppe direkte Bindung, -O-, -S(O)n-, -NR9-, -CR7=CR7-, -C/C-, -A1-, -A1-A1, -O-(A1)k-O-, -O-(A1)k-, -A3-, -A4-, -A10-, -A1S (O) n-, -A2-, OA2-, -NR9A2-, -OA2-A1-, -OA2-C(R7)=C(R8)-, -S(O)nA1-, -A1-A4-, -A1-A4-C(R8)=N-N=CR8-, -A1-A4-C(R8)=N-X2-X3-, -A1-A4-A3-, -A1-A4-N(R9)-, -A1-A4-X-CH2-, -A1-A4-A1-, -A1-A4-CH2X-, -A1-A4-C(R8)=N-X2-X3-X1-, -A1-X-C(R8)=N-, -A1-X-C(R8)=N-N=CR8-, -A1-X-C(R8)=N-N(R9)-, -A1-X-A-X1-, -A1-O-A3-, -A1-O-C(R7)=C(R8)-, -A1-O-N(R9)-A2-N-(R9)-, -A1-O-N (R9)-A2-, -A1-N(R9)-A2-N(R9)-, -A1-N(R9)-A2-,- A1-N(R9)-N=C(R8)-, -A3-A1-, -A4-A3-, -A2-NR9-, -A1-A2-X1-, -A1-A1-A2-X1-, -O-A2-N(R9)-A2-, -CR7=CR7-A2-X1-, -C/C-A2-X1-, -N=C(R8)-A2-X1-, -C(R8)=N-N=C (R8)-, -C(R8)=N-N(R9)-, -(CH2)2-O-N=C(R8)- und -X-A2-N(R9)-
wobei

n = 0, 1 oder 2,
k = 1 bis 9,
11 von 18
A1 = -CHR7-,
A2 = -C (=X) -,
A3 = -C(R8)=N-O-,
A4 = -O-N=C (R8) -,
X = O oder S,
X1 = O, S, NR9 oder eine direkte Bindung,
X2 = O, NR9 oder eine direkte Bindung,
X3 = Wasserstoff, -C(=O)-, -S02- oder eine direkte Bindung,
jedes R7 unabhängig ausgewählt ist aus der Gruppe unsubstituiertes oder substituiertes Alkyl, substituiertes oder unsubstituiertes Cycloalkyl, substituiertes oder unsubstituiertes Phenyl, Wasserstoff, Halogen, Cyano und Acyl;
jedes R8 unabhängig ausgewählt ist aus der Gruppe Alkyl, substituiertes oder unsubstituiertes Alkenyl, substituiertes oder unsubstituiertes Alkinyl, substituiertes oder unsubstituiertes Alkoxy, substituiertes oder unsubstituiertes Alkylthio, substituierte oder unsubstituierte carbocyclische oder heterocyclische einwertige Gruppe und Wasserstoff;
jedes R9 unabhängig ausgewählt ist aus der Gruppe unsubstituiertes oder substituiertes Alkyl, substituierte oder unsubstituierte einwertige carbocyclische oder heterocyclische Gruppe, und Acyl; oder zwei R9-Gruppen gemeinsam mit den sie verbindenden Atomen einen 5-7-gliedrigen Ring bilden können;

o die rechts von der Bindung A dargestellte Gruppe an $R^6$ gebunden ist oder -A-$R^6$ und $R^5$ gemeinsam mit dem Benzolring M ein System von unsubstituierten oder substituierten kondensierten Ringen bilden;

und von optischen und/oder geometrischen Isomeren, Tautomeren und Salzen von (I) mit einer pharmazeutisch unbedenklichen Säure oder Base;
und von Mischungen davon.

**2.** (geändert) Verwendung nach Anspruch 1, **dadurch gekennzeichnet, dass**

o $R^1$ ausgewählt ist aus der Gruppe Wasserstoff, substituiertes oder unsubstituiertes Alkyl, substituiertes oder unsubstituiertes Alkenyl und substituiertes oder unsubstituiertes Alkinyl, wobei, wenn $R^1$ substituiertes Alkyl, substituiertes Alkenyl oder substituiertes Alkinyl bedeutet, der Substituent bzw. die Substituenten davon ausgewählt ist/sind aus der Gruppe Alkoxy, Halogenalkoxy, Alkylthio, Halogen, unsubstituiertes Phenyl und Phenyl, das durch einen Rest aus der Gruppe Alkyl, Halogenalkyl, Alkoxy, Halogenalkoxy, Alkylthio und Halogen substituiert ist;
o $R^2$ und $R^3$ unabhängig ausgewählt sind aus der Gruppe R1, Alkoxy, Alkoxyalkyl, Benzyloxy, Cyano und Alkylcarbonyl;
o $R^4$ ausgewählt ist aus der Gruppe:

(a) substituiertes oder unsubstituiertes Alkyl, substituiertes oder unsubstituiertes Alkenyl und substituiertes oder unsubstituiertes Alkinyl, wobei, wenn $R^4$ substituiertes Alkyl, substituiertes Alkenyl oder substituiertes Alkinyl bedeutet, der Substituent bzw. die Substituenten davon ausgewählt ist/sind aus der Gruppe Alkoxy, Halogenalkoxy, Alkylthio, Halogen, unsubstituiertes Phenyl und Phenyl, das durch einen Rest aus der Gruppe Alkyl, Halogenalkyl, Alkoxy, Halogenalkoxy, Alkylthio und Halogen substituiert ist;
(b) Hydroxy;
(c) Halogen;
(d) Cyano;
(e) Acyl, Amin, Monoalkylamin, Dialkylamin, unsubstituiertes Phenyl und Phenyl, das durch einen Rest ausgewählt aus der Gruppe Alkyl, Halogenalkyl, Alkoxy, Halogenalkoxy und Alkylthio substituiert ist;

o m = 0 oder 1;
o $R^5$, falls vorhanden, eine Gruppe mit derselben Definition wie oben für $R^4$ angegeben ist,
o A eine direkte Bindung, -O-, -S-, -NR9-, -CHR7-oder -O-CHR7- bedeutet,
o jedes R9, wenn vorhanden, ausgewählt ist aus der Gruppe Wasserstoff, substituiertes oder unsubstituiertes Alkyl, substituiertes oder unsubstituiertes Alkenyl und substituiertes oder unsubstituiertes Alkinyl, wobei, wenn R9 substituiertes Alkyl, substituiertes Alkenyl oder substituiertes Alkinyl bedeutet, der Substituent bzw. die

Substituenten davon ausgewählt ist/sind aus der Gruppe Alkoxy, Halogenalkoxy, Alkylthio, Halogen, unsubstituiertes Phenyl und Phenyl, das durch einen Rest aus der Gruppe Alkyl, Halogenalkyl, Alkoxy, Halogenalkoxy, Alkylthio und Halogen substituiert ist;
o R7 ausgewählt ist aus der Gruppe R9, Hydroxy, Halogen, Cyano, Acyl, Alkoxy, Halogenalkoxy und Alkylthio,
o A an die 4-Stellung des Benzolrings M gebunden ist, und
o $R^6$ substituiertes oder unsubstituiertes Phenyl oder einen aromatischen Heterocyclus bedeutet, wobei, wenn $R^6$ ein substituiertes Phenyl oder einen substituierten aromatischen Heterocyclus bedeutet, der Substituent bzw. die Substituenten davon ausgewählt ist/sind aus der Gruppe

(a) Hydroxy;
(b) Halogen;
(c) Cyano;
(d) Acyl;
(e) Amin;
(f) Alkylamin;
(g) Dialkylamin;
(h) Alkyl;
(i) Halogenalkyl;
(j) RaO-Alkyl;
(k) Acyloxyalkyl;
(l) Cyanooxyalkyl;
(m) Alkoxy;
(n) Halogenalkoxy;
(o) Alkylthio;
(p) Cycloalkyl, unsubstituiert oder substituiert durch einen Rest, ausgewählt aus der Gruppe Alkyl, Halogenalkyl, Alkoxy, Halogenalkoxy und Alkylthio; und
(q) Benzyl, unsubstituiert oder substituiert durch einen Rest, ausgewählt aus der Gruppe Alkyl, Halogenalkyl, Alkoxy, Halogenalkoxy und Alkylthio.

**3.** (geändert) Verwendung nach Anspruch 1, **dadurch gekennzeichnet, dass**:

- $R^1$ ein Wasserstoffatom bedeutet
- $R^2$, $R^3$, $R^4$ und $R^5$ unabhängig $C_1$-$C_6$-Alkyl bedeuten und $R^5$ an den Kohlenstoff bei $C_5$ des Benzolrings M gebunden ist, wobei m = 1;
- A an den Kohlenstoff bei $C_4$ des Benzolrings M gebunden ist und -O- bedeutet;
- $R^6$ unsubstituiertes Aryl oder Aryl, das durch mindestens ein Alkyl und/oder mindestens ein Halogen substituiert ist, bedeutet.

**4.** (neu) Verwendung nach Anspruch 1, **dadurch gekennzeichnet, dass**:

- $R^1$ ein Wasserstoffatom bedeutet
- $R^2$ Ethyl bedeutet;
- $R^3$, $R^4$ und $R^5$ Methyl bedeuten und $R^5$ an den Kohlenstoff bei $C_5$ des Benzolrings M gebunden ist, wobei m = 1;
- A an den Kohlenstoff bei $C_4$ des Benzolrings M gebunden ist und -O- bedeutet;
- $R^6$ Benzyl, das durch mindestens ein Alkyl und/oder mindestens ein Halogen substituiert ist, bedeutet.

**5.** (geändert) Verwendung nach Anspruch 3 oder 4, **dadurch gekennzeichnet, dass** die Verbindung (I) ausgewählt ist aus der Gruppe:

- *N*-Ethyl-*N*-methyl-*N*-[4-(4-chlor-3-trifluormethylphenoxy)-2,5-dimethylphenyl]imidoformamid,
- *N*-Ethyl-*N*-methyl-*N*-[4-(4-Fluor-3-trifluormethylphenoxy)-2,5-dimethylphenyl]imidoformamid,
- *N*-Ethyl-*N*-methyl-*N*-[4-(4-cyano-3-trifluormethylphenoxy)-2,5-dimethylphenyl]imidoformamid,

und die möglichen Tautomere und Salze, insbesondere Additionssalze mit einer pharmazeutisch unbedenklichen Säure oder Base, dieser Verbindungen (I).

**6.** (geändert) Verwendung nach einem der Ansprüche 1 bis 5, **dadurch gekennzeichnet, dass** das Arzneimittel weiterhin mindestens eine andere antifungale Verbindung (II) umfasst.

**7.** (geändert) Verwendung nach Anspruch 6, **dadurch gekennzeichnet, dass** die antifungale Verbindung (II) ausgewählt ist aus der Gruppe Azole, Polyene, Allylamine, Benzylamine, Thiocarbamate, Candine, Nukleosidanaloge, Sordarine, Polyoxine, Nikkomycine, Pradimicine, Benanomycine, Aureobasidine, UK-2A oder UK-3A, kationische Peptide, allein oder als Mischung, sowie ihre möglichen Tautomere und Salze, insbesondere Additionssalze mit einer Säure oder einer Base, ihre Lipid- oder Liposomenformulierungen, die pharmazeutisch unbedenklich sind.

**8.** (neu) Verwendung nach Anspruch 7, **dadurch gekennzeichnet, dass** die antifungale Verbindung (II) ausgewählt ist aus der Gruppe Bifonazol, Butoconazol, Clotrimazol, Eberconazol, Econazol, Fenticonazol, Fluconazol, Iraconazol, Ketoconazol, Miconazol, Oxiconazol, Posaconazol, Sulconazol, Terconazol, Tioconazol, Voriconazol, Zinoconazol, Amphotericin B, Nystatin, Butenafin, Naftifin, Terbinafin, Tolnaftat, Caspofungin, Cilofungin, Flucytosin, die Nikkomycine Z, J, pseudo J, PX, RZ, pseudo Z, Pradimicin A; allein oder als Mischung, sowie ihre möglichen Tautomere und Salze, insbesondere Additionssalze mit einer Säure oder einer Base, ihre Lipid- oder Liposomenformulierungen, die pharmazeutisch unbedenklich sind.

**9.** (geändert) Verwendung nach den Ansprüchen 6 bis 8, **dadurch gekennzeichnet, dass** das Mengenverhältnis (I/II) $0,02 \leq I/II \leq 50$ beträgt.

**10.** (geändert) Verwendung nach einem der Ansprüche 1 bis 9, **dadurch gekennzeichnet, dass** das Arzneimittel weiterhin mindestens einen pharmazeutisch unbedenklichen Hilfsstoff umfasst.

**11.** (geändert) Verwendung nach einem der Ansprüche 1 bis 10, **dadurch gekennzeichnet, dass** das Arzneimittel 0,5 bis 99% der Kombination von Verbindung (I) und Verbindung (II) umfasst.

## Revendications

**1.** (modifiée) Utilisation, pour la fabrication d'un médicament destiné au traitement d'infections humaines par *Candida albicans* ou *Aspergillus fumigatus,* d'au moins un composé de formule (I) :

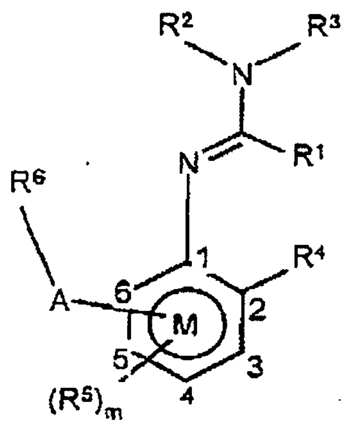

**(I)**

dans laquelle :

° $R^1$ est choisi dans le groupe constitué d'hydrogène, d'alkyle substitué ou non substitué, d'alcényle substitué ou non substitué, d'alcynyle substitué ou non substitué et d'un groupement monovalent carbocyclique ou hétérocyclique substitué ou non substitué ;

o $R^2$ et $R^3$ sont choisis indépendamment dans le groupe constitué de $R^1$ ; un cyano ; un acyle ; -ORa ou -SRa, où Ra est choisi dans le groupe constitué d'un alkyle substitué ou non substitué, d'un alcényle substitué ou non substitué, d'un alcynyle substitué ou non substitué, et d'un groupement monovalent carbocyclique ou hétérocyclique substitué ou non substitué ; ou

o $R^2$ et $R^3$, ou $R^2$ et $R^1$ forment ensemble et avec les atomes les reliant un cycle substitué ou non substitué ;

o $R^4$ est choisi dans le groupe constitué d'un alkyle substitué ou non substitué, un alcényle substitué ou non substitué, un alcynyle substitué ou non substitué, un groupement monovalent carbocyclique ou hétérocyclique substitué ou non substitué, hydroxy, mercapto, azido, nitro, halogéno, cyano, acyle non substitué ou substitué, amino, cyanato, thiocyanato, -SF5, -ORa, -SRa et -Si(Ra)3
o m = 0, 1, 2 ou 3 ;
o le groupement $R^5$ éventuel ou les groupements $R^5$ éventuels, qui peuvent être identiques ou différents entre eux, ont la même définition que celle donnée ci-dessus pour $R^4$ ;
o $R^6$ est un groupement carbocyclique ou hétérocyclique non substitué ou substitué ; et
o A est choisi dans le groupe constitué d'une liaison directe, -O-, -S(O)n-, -NR9-, - CR7=CR7-, -C/C-, -A1-, -A1-A1, -O-(A1)k-O-, - O-(A1)k-, -A3-, -A4-, -A10-, -A1S(O)n-, -A2-, OA2-, -NR9A2-, -OA2-A1-, -OA2-C(R7)=C (R8)-, - S(O)nA1-, -A1-A4-, -A1-A9-C(R8)=N-N=CR8-, -A1-A4-C(R8)=N-X2-X3-, -A1-A4-A3-, -A1-A4-N(R9)-, -A1-A4-X-CH2-, -A1-A4-A1-, -A1-A4-CH2X-, -A1-A4-C(R8)=N-X2-X3-X1-, -A1-X-C(R8)=N-, -A1-X-C(R8)=N-N=CR8-, -A1-X-C(R8)=N-N(R9)-, -A1-X-A-X1-, -A1-O-A3-, -A1-O-C(R7)=C(R8)-, -A1-O-N(R9)-A2-N-(R9)-, -A1-O-N (R9) -A2-, -A1-N (R9) -A2-N (R9) -, -A1-N (R9) - A2-, -A1-N(R9)-N=C(R8)-, -A3-A1-, -A4-A3-, - A2-NR9-, -A1-A2-X1-, -A1-A1-A2-X1-, -O-A2-N(R9)-A2-, -CR7=CR7-A2-X1-, -C/C-A2-X1-, - N=C(R8)-A2-X1-, -C (R8)=N-N=C(R8)-, -C(R8)=N-N(R9)-, -(CH2)2-O-N=C(R8)- et -X-A2-N(R9)-
où
n = 0, 1 ou 2,
k = 1 à 9,
11 de 18
A1 = -CHR7-,
A2 = -C(=X)-,
A3 = -C (R8 ) =N-O-,
A4 = -0-N=C(R8)-,
X = O ou S,
X1 = O, S, NR9 ou une liaison directe,
X2 = O, NR9 ou une liaison directe,
X3 = hydrogène, -C(=O)-, -S02- ou une liaison directe,
chaque R7 est choisi indépendamment dans le groupe constitué d'alkyle non substitué ou substitué, de cycloalkyle substitué ou non substitué, de phényle substitué ou non substitué, d'hydrogène, d'halogène, de cyano et d'acyle ;
chaque R8 est choisi indépendamment dans le groupe constitué d'alkyle, d'alcényle substitué ou non substitué, d'alcynyle substitué ou non substitué, d'alcoxy substitué ou non substitué, d'alkylthio substitué ou non substitué, d'un groupement monovalent carbocyclique ou hétérocyclique substitué ou non substitué, et d'hydrogène ;
chaque R9 est choisi indépendamment dans le groupe constitué d'alkyle non substitué ou substitué, d'un groupement carbocyclique ou hétérocyclique monovalent substitué ou non substitué, et d'acyle ; ou deux groupements R9 peuvent former ensemble, et avec les atomes les reliant, un cycle à 5-7 chaînons ;
o le groupement représenté au côté droit de la liaison A est lié à $R^6$ ; ou -A-$R^6$ et $R^5$ forment ensemble, avec le cycle benzène M, un système de cycles condensés non substitués ou substitués ;

et des isomères optiques et/ou géométriques, des tautomères et des sels de (I) avec un acide ou une base qui sont pharmaceutiquement acceptables ;
et des mélanges de ceux-ci.

**2.** (modifiée) Utilisation selon la revendication 1, **caractérisée en ce que** :

o $R^1$ est choisi dans le groupe constitué d'hydrogène, d'alkyle substitué ou non substitué, d'alcényle substitué ou non substitué et d'alcynyle substitué ou non substitué, où lorsque $R^1$ est alkyle substitué, alcényle substitué ou alcynyle substitué, le ou les substituants de ceux-ci sont choisis dans le groupe constitué d'alcoxy, halogénoalcoxy, alkylthio, halogène, phényle non substitué, et phényle substitué par un motif choisi dans le groupe constitué d'alkyle, halogénoalkyle, alcoxy, halogénoalcoxy, alkylthio et halogène ;
o $R^2$ et $R^3$ sont choisis indépendamment dans le groupe constitué de $R^1$, alcoxy, alcoxyalkyle, benzyloxy, cyano et alkylcarbonyle ;
o $R^9$ est choisi dans le groupe constitué de :

(a) alkyle substitué ou non substitué, alcényle substitué ou non substitué, alcynyle substitué ou non substitué, où lorsque $R^4$ est alkyle substitué, alcényle substitué ou alcynyle substitué, le ou les substituants de ceux-ci sont choisis dans le groupe constitué d'un alcoxy, halogénoalcoxy, alkylthio, un halogène, phényle non

substitué, et phényle substitué par un motif choisi dans le groupe constitué d'alkyle, halogénoalkyle, alcoxy, halogénoalcoxy, alkylthio et halogène ;
(b) hydroxy ;
(c) halogène ;
(d) cyano ;
(e) acyle, amine, monoalkylamine, dialkylamine, phényle non substitué, et phényle substitué par un motif choisi dans le groupe constitué d'alkyle, halogénoalkyle, alcoxy, halogénoalcoxy et alkylthio ;

o m = 0 ou 1 ;
o lorsqu'il est présent, $R^5$ est un groupement ayant la même définition que celle donnée ci-dessus pour $R^4$,
o A est une liaison directe, -O-, -S-, -NR9-, - CHR7- ou -O-CHR7-,
o chaque R9, lorsqu'il en existe, est choisi dans le groupe constitué d'hydrogène, d'alkyle substitué ou non substitué, d'alcényle substitué ou non substitué et d'alcynyle substitué ou non substitué, où lorsqu'un R9 est un alkyle substitué, un alcényle substitué ou un alcynyle substitué, le ou les substituants de ceux-ci sont choisis dans le groupe constitué d'alcoxy, halogénoalcoxy, alkylthio, halogène, phényle non substitué, et phényle substitué par un motif choisi dans le groupe constitué d'alkyle, halogénoalkyle, alcoxy, halogénoalcoxy, alkylthio et halogène ;
o R7 est choisi dans le groupe constitué de R9 ; hydroxy ; halogène ; cyano ; acyle ; alcoxy ; halogénoalcoxy ; et alkylthio ;
o A est lié en position 4 du cycle benzène M ; et
o $R^6$ est un phényle substitué ou non substitué ou un hétérocycle aromatique où lorsque $R^6$ est un phényle substitué ou un hétérocycle aromatique substitué, le ou les substituants de celui-ci sont choisis dans le groupe constitué de

(a) hydroxy ;
(b) halogène ;
(c) cyano ;
(d) acyle ;
(e) amine ;
(f) alkylamine ;
(g) dialkylamine ;
(h) alkyle ;
(i) halogénoalkyle ;
(j) RaO-alkyle ;
(k) acyloxyalkyle ;
(l) cyanooxyalkyle ;
(m) alcoxy ;
(n) halogénoalcoxy ;
(o) alkylthio ;
(p) cycloalkyle non substitué ou substitué par un motif choisi dans le groupe constitué d'alkyle, halogénoalkyle, alcoxy, halogénoalcoxy et alkylthio ; et
(q) benzyle non substitué ou substitué par un motif choisi dans le groupe constitué d'alkyle, halogénoalkyle, alcoxy, halogénoalcoxy et alkylthio.

**3.** (modifiée) Utilisation selon la revendication 1, **caractérisée en ce que**

- $R^1$ représente un atome d'hydrogène
- $R^2$, $R^3$, $R^4$ et $R^5$ représentent indépendamment alkyle en $C_1$-$C_6$ et $R^5$ est lié au carbone à $C_5$ du cycle benzyle M, avec m = 1 ;
- A est lié au carbone à $C_4$ du cycle benzyle M et représente -O- ;
- $R^6$ représente aryle non substitué ou aryle substitué par au moins un alkyle et/ou par au moins un halogène.

**4.** (Nouvelle) Utilisation selon la revendication 1, **caractérisée en ce que** :

- $R^1$ représente un atome d'hydrogène ;
- $R^2$ représente éthyle ;
- $R^3$, $R^4$ et $R^5$ représentent méthyle et $R^5$ est lié au carbone à $C_5$ du cycle benzyle M, avec m = 1 ;
- A est lié au carbone à $C_4$ du cycle benzyle M et représente -O- ;

- $R^6$ représente benzyle substitué par au moins un alkyle et/ou par au moins un halogène.

**5.** (modifiée) Utilisation selon la revendication 3 ou 4, **caractérisée en ce que** le composé (I) est choisi dans le groupe constitué de :

- *N*-éthyl-*N*-méthyl-*N'*-[4-(4-chloro-3-trifluorométhylphénoxy)-2,5-diméthylphényl]imidoformamide,
- *N*-éthyl-*N*-méthyl-*N'*-[4-(4-fluoro-3-trifluorométhylphénoxy)-2,5-diméthylphényl]imidoformamide,
- *N*-éthyl-*N*-méthyl-*N'*-[4-(4-cyano-3-trifluorométhylphénoxy)-2,5-diméthylphényl]imidoformamide,

et des tautomères et sels possibles, en particulier des sels d'addition avec un acide ou une base, qui sont pharmaceutiquement acceptables, de ces composés (I).

**6.** (modifiée) Utilisation selon l'une des revendications 1 à 5, **caractérisée en ce que** le médicament comprend en outre au moins un autre composé (II) antifongique.

**7.** (modifiée) Utilisation selon la revendication 6, **caractérisée en ce que** le composé (II) antifongique est choisi dans le groupe constitué d'azoles ; polyènes ; allylamines, benzylamines ; thiocarbamates ; candines ; analogues nucléosidiques ; sordarines ; polyoxines, nikkomycines ; pradimicines ; bénanomycines ; auréobasidines ; UK-2A ou UK-3A ; peptides cationiques ; pris ensemble ou en mélange, et leurs tautomères et sels possibles, en particulier les sels d'addition avec un acide ou une base, leurs formulations lipidiques ou liposomales, qui sont pharmaceutiquement acceptables.

**8.** (nouvelle) Utilisation selon la revendication 7, **caractérisée en ce que** le composé (II) antifongique est choisi dans le groupe constitué de bifonazole, butoconazole, clotrimazole, éberconazole, éconazole, fenticonazole, fluconazole, itraconazole, kétoconazole, miconazole, oxiconazole, posaconazole, sulconazole, terconazole, tioconazole, voriconazole, zinoconazole, amphotéricine B, nystatine, buténafine, naftifine, terbinafine, tolnaftate, caspofungine, cilofungine, flucytosine, nikkomycines Z, J, pseudo J, PX, RZ, pseudo Z, pradimicine A ; pris ensemble ou en mélange, et leurs tautomères et sels possibles, en particulier les sels d'addition avec un acide ou une base, leurs formulations lipidiques ou liposomales, qui sont pharmaceutiquement acceptables.

**9.** (modifiée) Utilisation selon les revendications 6 à 8, **caractérisée en ce que** le rapport en masse (I/II) est $0,02 \leq I/II \leq 50$.

**10.** (modifiée) Utilisation selon l'une des revendications 1 à 9, **caractérisée en ce que** le médicament comprend en outre au moins un excipient pharmaceutiquement acceptable.

**11.** (modifiée) Utilisation selon l'une des revendications 1 à 10, **caractérisée en ce que** le médicament comprend de 0,5 à 99 % de la combinaison de composé (I) et de composé (II).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0046184 A


### Non-patent literature cited in the description


- **U. Gisi.** Synergistic interaction in fungicide mixtures. *Phytopathology,* vol. 86, 1273-1279